# EUROPEAN PATENT APPLICATION

(11) **EP 4 311 861 A1**
(43) Date of publication of application: **31.01.2024**
(21) Application number: 22187343.3
(22) Date of filing: 27.07.2022
(51) Int. Cl.: C12Q 1/6816

(54) **DNA-STABILIZED METAL CLUSTER-BASED NUCLEIC ACID-SENSOR**

(71) Applicant: Fraunhofer-Gesellschaft zur Förderung der angewandten Forschung e.V., 80686 München (DE)
(72) Inventor: Hoffmann, Nicolas, 97070 Würzburg (DE); Witzel, Patrick, 97074 Würzburg (DE); Meinhardt, Jürgen, 97450 Arnstein (DE); Heinrich, Doris, 63875 Mespelbrunn (DE)
(74) Representative: Ulrich, Thomas Franz

(57) **Abstract**

The present invention pertains to a DNA-stabilized metal cluster-based nucleic acid-sensor which allows quantitative and qualitative detection of oligo- or polynucleotides by measuring a shift in fluorescence intensity between distinct emission wavelengths triggered by target recognition. The invention further pertains to method for detecting a target polynucleotide with a nucleic acid-sensor according to the present invention.

## Description

The present invention pertains to a DNA-stabilized metal cluster-based nucleic acid-sensor, which allows quantitative and qualitative detection of polynucleotides by measuring a shift in fluorescence intensity between distinct emission wavelengths triggered by target recognition. The invention further pertains to a method for detecting a target polynucleotide with a nucleic acid-sensor according to the present invention. Particularly, the DNA-stabilized metal cluster-based nucleic acid-sensor according to the present invention is a silver quantum cluster-based sensor allowing for the label- and amplification-free detection of target polynucleotides. The sensing functionality is based on a change of the ratio between the fluorescence intensities at distinct wavelengths upon hybridization of a target nucleotide sequence and the nucleic acid-sensor.

Polynucleotides are small sections of genetic material that can be used as an indicator for the presence or absence of toxic or potentially dangerous organisms in an environment. The detection and quantification of specific polynucleotides has become an import tool for a wide range of medical and non-medical applications, such as diagnosis, disease monitoring, sample analytics as well as for quality control to ensure food and feed safety. The increasingly relevant field of home and Point-of-Care (PoC) testing poses new challenges for existing analytical methods, such as PCR, some of which can only be solved by establishing novel techniques.

The central requirements for analytical methods are sensitivity, specificity and reliability. Apart from these requirements, the analytical methods are further desired to allow rapid target detection, to be cost-efficient, simple and user-friendly as well as to also be applicable outside a laboratory environment.

Currently a number of different methods for the detection and quantification of polynucleotides is known. The current "gold-standard" for the qualitative and quantitative detection of target nucleotides is RTD-PCR/qPCR (Real-Time Quantitative Polymerase Chain Reaction). The method is based on the specific amplification of a target polynucleotide and a quantification of the PCR product during each cycle by determining a corresponding increase in fluorescence signal. As the number of amplicons detected in the exponential phase of the PCR amplification is directly proportional to the initial number of target nucleotides, RTD-PCR/qPCR allows calculation of the initial number of target sequences in a sample. The major disadvantage of RTD-PCR/qPCR is that it requires specialized trained operators and a laboratory environment. Furthermore, the time required for obtaining results is usually 1 to 3 hours.

A rapidly expanding research field is the use of fluorescent DNA-stabilized quantum/nano metal cluster (QC-DNA) oligo- or polynucleotide biosensors. These kind of oligo- or polynucleotide biosensors can generally be categorized in quenching-based QC-DNA biosensors, enhancer-based QC-DNA biosensors, shift-based QC-DNA biosensors and recovery-based QC-DNA biosensors.

Quenching-based methods are based on the (reversible) quenching of the fluorescent signal of a fluorescent unit. The quenching is controlled by the spatial proximity of the fluorescent and quenching units. The target recognition induces a change in the spatial proximity of the fluorescent and quenching units which either enables or disables the quenching effect. This results in a measurable decrease or increase in the general intensity of the fluorescence emitted by the fluorescent unit. Thus, the detection is based on a change in signal intensity at a given wavelength.

Similarly, enhancer-based methods are based on the (reversible) amplification of a given fluorescent signal of a fluorescent unit. An enhancing unit of the biosensor increases the fluorescent signal of the fluorescent unit dependent on the spatial proximity of the two units with respect to one another. A change in the spatial proximity triggered by target recognition results in either an increase or decrease in the general intensity of the fluorescent signal.

In contrast thereto, shift-based QC-DNA methods are based on a (reversible) shift in wavelength of the fluorescence signal of the fluorescent unit. In this kind of sensors, a structural change within or in the immediate proximity of the fluorescent unit is triggered by target recognition. The structural change is associated with a measurable shift in the fluorescent signal to either higher or lower wavelengths. Shift-based QC-DNA sensors are usually rather complex structures composed of at least three DNA structures.

A further group are so-called recovery-based QC-DNA sensors, which comprise an oligo- or polynucleotide hairpin-structure with a silver nanocluster in the hairpin loop. In the absence of the target oligo- or polynucleotide, the constructs usually emit red fluorescence. Once the target nucleotide is bound, a green fluorescence signal can be detected in a concentration-dependent manner.

The present invention overcomes the disadvantages associated with the methods known in the prior art and in particular provides a DNA-stabilized metal cluster-based nucleic acid-sensor as well as a method for detecting and quantifying a target polynucleotide employing the DNA-stabilized metal cluster-based nucleic acid-sensor according to the present invention.

The technical problem underlying the present invention is solved by the subject-matter of the independent claims.

The present invention in particular pertains to a DNA-stabilized metal cluster-based nucleic acid-sensor, comprising:
a) at least one template nucleotide sequence for silver quantum cluster (AgQC) formation,
b) at least one target-recognition nucleotide sequence, and
c) at least one nucleotide sequence for intra-molecular hybridization,
   wherein the DNA-stabilized metal cluster-based nucleic acid-sensor comprises a stem-loop structure in the absence of a target nucleotide sequence.

Thus, the nucleic acid-sensor according to the present invention comprises three major functional units. The at least one template nucleotide sequence of the sensor functions to bind and stabilize a silver quantum cluster (AgQC). Quantum clusters (QC) of noble metals are a class of materials with a core dimension of less than 1 nm. DNA-templated silver quantum cluster formation was first reported in 2004 to have strong characteristic electronic transitions between about 400 and 600 nm. The at least one target-recognition nucleotide sequence of the nucleic acid-sensor according to the present invention comprises a nucleotide sequence which is complementary to the sequence of a target polynucleotide to be detected. Apart from this, the nucleic acid-sensors according to the present invention are characterized by comprising at least one nucleotide sequence for intra-molecular hybridization, wherein the intra-molecular hybridization results in the formation of stem-loop structure in the absence of a target nucleotide sequence. Once a target nucleotide sequence hybridizes with the at least one target-recognition nucleotide sequence of the sensor, a conformational change is triggered which either results in the dissolution of the intra-molecular hybridization, in particular of the stem-loop structure, or solely leads to a change of the quantum cluster's environment while maintaining the stem-loop structure of the nucleic acid-sensor. In both cases, a measurable shift in fluorescence intensity between two characteristic wavelengths in the fluorescence spectrum of the nucleic acid-sensor according to the present invention occurs.

Accordingly, the nucleic acid-sensors of the present invention are composed of simple nucleotides and chemicals and do not require complex and cost-intensive modification. Apart from their relatively simple structure and cost-efficient preparation, the nucleic acid-sensors of the present invention are advantageously characterized by high sensitivity and specificity for the target polynucleotide. The dispensability of a laboratory environment for performing and evaluating target detection opens a wide range of applications for the nucleic acid-sensors of the present invention, such as clinical or Point-of-Care (PoC) detection of bacteria or viruses, sensing of polynucleotides within living cells for target imaging, etc. The principle underlying target recognition and quantification using the nucleic acid-sensors according to the present invention is based on a shift in the fluorescence intensity between two characteristic fluorescence maxima in the fluorescence spectrum. In contrast to conventional fluorescence-based on/off-switch sensors which enhance, quench or shift a given fluorescence signal, the nucleic acid-sensors according to the present invention allow to easily assess the "readiness for use" and the sensors integrity before detection of the target polynucleotide. The measurement of two particular peaks in a fluorescence spectrum and the determination of the ratio between the fluorescence intensity of the signals for target detection and quantification makes the method less sensitive to the concentration of the sensors and increases the accuracy, in particular under conditions where some of the sensors degrade or where the sensor concentration is not uniform. The simple and rapid detection of the shift in signal intensity between two characteristic peaks in the fluorescence spectrum of the nucleic acid-sensors according to the present invention further allows for conducting target detection and quantification outside the environment of a qualified laboratory and enables their application at home and PoC testing.

According to a preferred embodiment of the present invention, the nucleotide sequences of a), b), and c) are part of a single nucleotide strand.

In a preferred embodiment, the nucleotide sequences of a), b), and c) are part of a single nucleotide strand which has a length of at least 20 nucleotides, preferably least 25 nucleotides, preferably at least 30 nucleotides, preferably at least 35 nucleotides, preferably least 40 nucleotides, preferably at least 45 nucleotides, preferably at least 50 nucleotides, preferably at least 55 nucleotides, preferably at least 60 nucleotides, preferably at least 65 nucleotides, preferably at least 70 nucleotides, preferably at least 75 nucleotides, preferably at least 80 nucleotides.

Preferably, the nucleotide sequences of a), b), and c) are part of a single nucleotide strand which has a length of at most 100 nucleotides, preferably at most 95 nucleotides, preferably at most 90 nucleotides, preferably at most 85 nucleotides, preferably at most 80 nucleotides, preferably at most 75 nucleotides, preferably at most 70 nucleotides, preferably at most 65 nucleotides, preferably at most 60 nucleotides, preferably at most 55 nucleotides, preferably at most 50 nucleotides.

According to a preferred embodiment of the present invention, the nucleotide sequences of a), b), and c) are part of a single nucleotide strand which has a length of 20 to 100 nucleotides, preferably 25 to 95 nucleotides, preferably 30 to 90 nucleotides, preferably 35 to 85 nucleotides, preferably 40 to 80 nucleotides, preferably 45 to 75 nucleotides, preferably 50 to 70 nucleotides, preferably 50 to 60 nucleotides, most preferably 52 to 58 nucleotides.

In a preferred embodiment of the present invention, the at least one template nucleotide sequence for silver quantum cluster (AgQC) formation has a length of at least 10 nucleotides, preferably at least 11 nucleotides, preferably at least 12 nucleotides, preferably at least 13 nucleotides, preferably at least 14 nucleotides, preferably at least 15, preferably at least 16 nucleotides, preferably at least 17 nucleotides, preferably at least 18 nucleotides, preferably at least 19 nucleotides, preferably at least 20 nucleotides.

Preferably, the at least one template nucleotide sequence for silver quantum cluster (AgQC) formation has a length of at most 35 nucleotides, preferably at most 34 nucleotides, preferably at most 33 nucleotides, preferably at most 32 nucleotides, preferably at most 31 nucleotides, preferably at most 30 nucleotides, preferably at most 28 nucleotides, preferably at most 26 nucleotides, preferably at most 24 nucleotides, preferably at most 22 nucleotides, preferably at most 20 nucleotides, preferably at most 18 nucleotides, preferably at most 16 nucleotides, preferably at most 14 nucleotides, preferably at most 12 nucleotides, preferably at most 10 nucleotides.

According to a preferred embodiment, the at least one template nucleotide sequence for silver quantum cluster (AgQC) formation has a length of 10 to 35 nucleotides, preferably 12 to 30 nucleotides, preferably 15 to 25 nucleotides, preferably 20 to 24 nucleotides.

In a particularly preferred embodiment of the present invention, the at least one template nucleotide sequence for silver quantum cluster (AgQC) formation comprises at least 25% cytosine, preferably at least 30% cytosine, preferably at least 35% cytosine, preferably at least 40% cytosine, preferably at least 45% cytosine, preferably at least 50% cytosine, preferably at least 55% cytosine, preferably at least 60% cytosine, preferably at least 65% cytosine, preferably at least 70% cytosine, preferably at least 75% cytosine, preferably at least 80% cytosine, preferably at least 85% cytosine, preferably at least 90% cytosine, preferably at least 95% cytosine (based on the total nucleotides of the template nucleotide sequence for silver quantum cluster (AgQC) formation).

Preferably, the at least one template nucleotide sequence for silver quantum cluster (AgQC) formation comprises 20 to 100% cytosine, preferably 25 to 95% cytosine, preferably 30 to 90% cytosine, preferably 35 to 85% cytosine, preferably 40 to 80% cytosine, preferably 45 to 75% cytosine, preferably 50 to 70% cytosine, preferably 55 to 65% cytosine (based on the total nucleotides of the template nucleotide sequence for silver quantum cluster (AgQC) formation).

Particularly preferred, the DNA-stabilized metal cluster-based nucleic acid-sensor comprises at least one silver quantum cluster (AgQC).

In a preferred embodiment of the present invention, the at least one silver quantum cluster (AgQC) comprises at least 3 silver atoms, preferably at least 4 silver atoms, preferably at least 5 silver atoms, preferably at least 6 silver atoms, preferably at least 7 silver atoms, preferably at least 8 silver atoms, preferably at least 9 silver atoms, preferably at least 10 silver atoms, preferably at least 12 silver atoms, preferably at least 14 silver atoms, preferably at least 16 silver atoms, preferably at least 18 silver atoms, preferably at least 20 silver atoms, preferably at least 22 silver atoms, preferably at least 24 silver atoms, preferably at least 25 silver atoms.

According to a preferred embodiment of the present invention, the at least one silver quantum cluster (AgQC) has a size, preferably an average size, of at most 40 silver atoms, preferably at most 35 silver atoms, preferably at most 30 silver atoms, preferably at most 25 silver atoms, preferably at most 20 silver atoms, preferably at most 18 silver atoms, preferably at most 16 silver atoms, preferably at most 14 silver atoms, preferably at most 12 silver atoms, preferably at most 10 silver atoms, preferably at most 8 silver atoms, preferably at most 6 silver atoms.

In a particularly preferred embodiment of the present invention, the at least one silver quantum cluster (AgQC) comprises 3 to 30 silver atoms, preferably 4 to 25 silver atoms, preferably 5 to 20 silver atoms, preferably 8 to 15 silver atoms.

In a further preferred embodiment of the present invention, the at least one nucleotide sequence for intra-molecular hybridization comprises:
i) at least one nucleotide sequence which is partially complementary to the target-recognition sequence, preferably to the 5'-end of the target-recognition sequence, and
ii) at least one nucleotide sequence which is partially complementary to the template sequence for silver quantum cluster (AgQC) formation, preferably to the 3'-end of the template sequence for silver quantum cluster (AgQC) formation.

Thus, in a particular aspect, the present invention pertains to a DNA-stabilized metal cluster-based nucleic acid-sensor comprising:
a) at least one template nucleotide sequence for silver quantum cluster (AgQC) formation,
b) at least one target-recognition nucleotide sequence,
c1) at least one nucleotide sequence which is partially complementary to the target-recognition sequence, preferably to the 5'-end of the target-recognition sequence, and
c2) at least one nucleotide sequence which is partially complementary to the template sequence for silver quantum cluster (AgQC) formation, preferably to the 3'-end of the template sequence for silver quantum cluster (AgQC) formation,
   wherein the DNA-stabilized metal cluster-based nucleic acid-sensor comprises a stem-loop structure in the absence of a target nucleotide sequence.

According to this preferred embodiment of the present invention, i) the at least one template nucleotide sequence for silver quantum cluster (AgQC) formation and the at least one nucleotide sequence which is partially complementary to the template sequence for silver quantum cluster (AgQC) formation, preferably to the 3'-end of the template sequence for silver quantum cluster (AgQC) formation, as well as ii) the at least one target-recognition nucleotide sequence and the at least one nucleotide sequence which is partially complementary to the target-recognition sequence, preferably to the 5'-end of the target-recognition sequence, hybridize to form a closed stem-loop structure. In a particular aspect of the present invention, this conformation of the DNA-stabilized metal cluster-based nucleic acid-sensor constitutes the "ready-to-use" closed state.

In case of target recognition, the target nucleotide sequence hybridizes with the at least one target-recognition nucleotide sequence, thereby dissolving the hybridization between i) the at least one nucleotide sequence which is partially complementary to the target-recognition nucleotide sequence, preferably to the 5'-end of the target-recognition nucleotide sequence, and the at least one target-recognition nucleotide sequence as well as between ii) the at least one nucleotide sequence which is partially complementary to the template sequence for silver quantum cluster (AgQC) formation, preferably to the 3'-end of the template sequence for silver quantum cluster (AgQC) formation and the at least one template nucleotide sequence for silver quantum cluster (AgQC) formation.

According to this embodiment, the conformational change triggered by hybridization of the target nucleotide sequence and the at least one target-recognition nucleotide sequence dissolves the closed stem-loop structure and associated therewith results in a shift in the fluorescence intensity at the excitation and emission wavelengths of the DNA-stabilized metal cluster-based nucleic acid-sensor according to the present invention.

In a preferred embodiment of the present invention, the at least one target-recognition nucleotide sequence has a length of at least 10 nucleotides, preferably at least 11 nucleotides, preferably at least 12 nucleotides, preferably at least 14 nucleotides, preferably least 15 nucleotides, preferably at least 16 nucleotides, preferably at least 17 nucleotides, preferably at least 18 nucleotides, preferably at least 19 nucleotides, preferably at least 20 nucleotides, preferably at least 21 nucleotides, preferably at least 22 nucleotides, preferably at least 23 nucleotides, preferably at least 24, preferably at least 25 nucleotides, preferably at least 26 nucleotides, preferably at least 27 nucleotides, preferably at least 28, preferably at least 29 nucleotides, preferably at least 30 nucleotides.

Preferably, the at least one target-recognition nucleotide sequence has a length of at most 80 nucleotides, preferably at most 70 nucleotides, preferably at most 60 nucleotides, preferably at most 50 nucleotides, preferably at most 45 nucleotides, preferably at most 40 nucleotides, preferably at most 35 nucleotides, preferably at most 30 nucleotides, preferably at most 25 nucleotides.

In a particularly preferred embodiment of the present invention, the at least one target-recognition nucleotide sequence has a length of 10 to 80 nucleotides, preferably 11 to 60 nucleotides, preferably 12 to 50 nucleotides, preferably 14 to 45 nucleotides, preferably 16 to 40 nucleotides, preferably 18 to 35 nucleotides, preferably 20 to 25 nucleotides.

According to a further preferred embodiment of the present invention, the at least one target-recognition nucleotide sequence of the DNA-stabilized metal cluster-based nucleic acid-sensor is composed of at least two partial target-recognition nucleotide sequences. Preferably, the at least one target-recognition nucleotide sequence of the DNA-stabilized metal cluster-based nucleic acid-sensor is composed of at most two partial target-recognition nucleotide sequences. Particularly preferred, the at least one target-recognition nucleotide sequence of the DNA-stabilized metal cluster-based nucleic acid-sensor is composed of exactly two partial target-recognition nucleotide sequences.

In a preferred embodiment, the partial target-recognition nucleotide sequences, in particular the two partial target-recognition nucleotide sequences, together form a target-recognition sequence suitable to hybridize with a complementary target nucleotide sequence.

Thus, a particular aspect of the present invention pertains to a DNA-stabilized metal cluster-based nucleic acid-sensor comprising:
a) at least one template nucleotide sequence for silver quantum cluster (AgQC) formation,
b1) at least two partial target-recognition nucleotide sequences, and
c) at least one nucleotide sequence for intra-molecular hybridization,
   wherein the DNA-stabilized metal cluster-based nucleic acid-sensor comprises a stem-loop structure in the absence of a target nucleotide sequence.

In a preferred embodiment of the present invention, the at least two partial target-recognition nucleotide sequences are located at different locations of a single nucleotide strand.

In a particularly preferred embodiment of the present invention, the at least two partial target-recognition nucleotide sequences are located on a single nucleotide strand and are separated from one another by at least 15 nucleotides, preferably at least 16 nucleotides, preferably at least 17 nucleotides, preferably at least 18 nucleotides, preferably at least 19 nucleotides, preferably at least 20 nucleotides, preferably at least 21 nucleotides, preferably at least 22 nucleotides, preferably at least 23 nucleotides, preferably at least 24 nucleotides, preferably at least 25 nucleotides, preferably at least 26 nucleotides, preferably at least 27 nucleotides, preferably at least 28 nucleotides, preferably at least 29 nucleotides, preferably at least 30 nucleotides, preferably at least 31 nucleotides, preferably at least 32 nucleotides, preferably at least 33 nucleotides, preferably at least 34 nucleotides, preferably at least 35 nucleotides.

In a further preferred embodiment, the at least two partial target-recognition nucleotide sequences are located on a single nucleotide strand and are separated from one another by at most 80 nucleotides, preferably at most 75 nucleotides, preferably at most 70 nucleotides, preferably at most 65 nucleotides, preferably at most 60 nucleotides, preferably at most 55 nucleotides, preferably at most 50 nucleotides, preferably at most 45 nucleotides, preferably at most 40 nucleotides.

Particularly preferred, the at least two partial target-recognition nucleotide sequences are located on a single nucleotide strand and are separated from one another by 15 to 80 nucleotides, preferably 20 to 60 nucleotides, preferably 25 to 50 nucleotides, preferably 30 to 45 nucleotides, preferably 35 to 40 nucleotides.

According to a preferred embodiment of the present invention, the at least two partial target-recognition nucleotide sequences are located at the opposite ends of the single nucleotide strand.

Preferably, at least one partial target-recognition nucleotide sequence is located in the 5' region of the single nucleotide strand and at least one partial target-recognition nucleotide sequence is located in the 3' region of the single nucleotide strand.

In a preferred embodiment of the present invention, at least one partial target-recognition nucleotide sequence is located at a distance less than 15 nucleotides, preferably less than 10 nucleotides, preferably less than 8 nucleotides, preferably less than 6 nucleotides, preferably less than 4 nucleotides, from the 5'-end of the single nucleotide strand.

Preferably, at least one partial target-recognition nucleotide sequence is located at the 5'-end of the single nucleotide strand.

Preferably, at least one partial target-recognition nucleotide sequence is located at a distance less than 15 nucleotides, preferably less than 10 nucleotides, preferably less than 8 nucleotides, preferably less than 6 nucleotides, preferably less than 4 nucleotides, preferably less than 3 nucleotides, preferably less than 2 nucleotides, from the 3'-end of the single nucleotide strand.

Preferably, at least one partial target-recognition nucleotide sequence is located at the 3'-end of the single nucleotide strand.

In a further preferred embodiment of the present invention, the DNA-stabilized metal cluster-based nucleic acid-sensor comprises two partial target-recognition nucleotide sequence, wherein a first partial target-recognition nucleotide sequence is located at a distance less than 15 nucleotides, preferably less than 10 nucleotides, preferably less than 8 nucleotides, preferably less than 6 nucleotides, preferably less than 4 nucleotides, preferably less than 3 nucleotides, preferably less than 2 nucleotides, from the 5'-end of the single nucleotide strand, preferably is located at the 5'-end of the single nucleotide strand, and wherein a second partial target-recognition nucleotide sequence is located at a distance less than 15 nucleotides, preferably less than 10 nucleotides, preferably less than 8 nucleotides, preferably less than 6 nucleotides, preferably less than 4 nucleotides, preferably less than 3 nucleotides, preferably less than 2 nucleotides, from the 3'-end of the single nucleotide strand, preferably is located at the 3'-end of the single nucleotide strand.

Particularly preferred, the DNA-stabilized metal cluster-based nucleic acid-sensor comprises two partial target-recognition nucleotide sequence, wherein a first partial target-recognition nucleotide sequence is located at the 5'-end of the single nucleotide strand, and wherein a second partial target-recognition nucleotide sequence is located at the 3'-end of the single nucleotide strand.

Preferably, a single partial target-recognition nucleotide sequences makes up at least 10%, preferably at least 15%, preferably at least 20%, preferably at least 25%, preferably at least 30%, preferably at least 35%, preferably at least 40%, preferably at least 45%, of the target-recognition nucleotide sequence.

In a preferred embodiment, a single partial target-recognition nucleotide sequences makes up at most 90%, preferably at most 85%, preferably at most 75%, preferably at most 70%, preferably at most 65%, preferably at most 60%, preferably at most 55%, of the target-recognition nucleotide sequence.

According to the specific embodiment of the present invention, in which the at least one target-recognition nucleotide sequence of the DNA-stabilized metal cluster-based nucleic acid-sensor is composed of at least two partial target-recognition nucleotide sequences, the at least one template nucleotide sequence for silver quantum cluster (AgQC) formation comprises a stem-loop structure.

According to a particularly preferred embodiment of the present invention, the at least one template nucleotide sequence for silver quantum cluster (AgQC) formation comprises a stem-loop structure. Preferably, the stem-loop structure is stabilized by the at least one nucleotide sequence for intra-molecular hybridization.

In a preferred embodiment of the present invention, the at least one template nucleotide sequence for silver quantum cluster (AgQC) formation comprises a stem-loop structure. Preferably, the stem-loop structure is stabilized by the at least one nucleotide sequence for intra-molecular hybridization, and at least two partial target-recognition nucleotide sequences are located in the 5'- and 3'-region of a single nucleotide strand, in particular are located at the 5'- and 3'-end of a single nucleotide strand, of the DNA-stabilized metal cluster-based nucleic acid-sensor. This conformation of the DNA-stabilized metal cluster-based nucleic acid-sensor constitutes the "ready-to-use" state in this particular aspect of the present invention.

In case of target recognition, the target nucleotide sequence hybridizes with the at least two target-recognition nucleotide sequences, thereby triggering a structural change resulting in a shift in the fluorescence intensity at particular excitation and emission wavelengths of the DNA-stabilized metal cluster-based nucleic acid-sensor according to the present invention.

According to a preferred embodiment of the present invention, the at least one template nucleotide sequence for silver quantum cluster (AgQC) formation comprises at least two, preferably two, nucleotide sequences for intra-molecular hybridization.

Preferably, the at least two, preferably two, nucleotide sequences for intra-molecular hybridization are located on a single nucleotide strand. Preferably, the at least two, preferably two, nucleotide sequences for intra-molecular hybridization are located within the at least one template nucleotide sequence for silver quantum cluster (AgQC) formation.

Particularly preferred, the at least two, preferably two, nucleotide sequences for intra-molecular hybridization are located in different locations on a single nucleotide strand and hybridize to trigger formation of a stem-loop structure. Preferably, the at least two, preferably two, nucleotide sequences for intra-molecular hybridization are located in different locations within the at least one template nucleotide sequence for silver quantum cluster (AgQC) formation and hybridize to trigger formation of a stem-loop structure.

In a further preferred embodiment of the present invention, the at least two, preferably two, nucleotide sequences for intra-molecular hybridization are located on a single nucleotide strand, preferably are located within the at least one template nucleotide sequence for silver quantum cluster (AgQC) formation, and are separated from one another by at least 5 nucleotides, preferably at least 6 nucleotides, preferably at least 7 nucleotides, preferably at least 8 nucleotides, preferably at least 9 nucleotides, preferably at least 10 nucleotides, preferably at least 11 nucleotides.

Preferably, the at least two, preferably two, nucleotide sequences for intra-molecular hybridization are located on a single nucleotide strand, preferably are located within the at least one template nucleotide sequence for silver quantum cluster (AgQC) formation, and are separated from one another by at most 30 nucleotides, preferably at most 25 nucleotides, preferably at most 20 nucleotides, preferably at most 15 nucleotides, preferably at most 14 nucleotides, preferably at most 13 nucleotides, preferably at most 12 nucleotides.

In to a preferred embodiment of the present invention, the at least two, preferably two, nucleotide sequences for intra-molecular hybridization are located on a single nucleotide strand, preferably are located within the at least one template nucleotide sequence for silver quantum cluster (AgQC) formation, and are separated from one another by 5 to 30 nucleotides, preferably 6 to 25 nucleotides, preferably 7 to 20 nucleotides, preferably 8 to 12 nucleotides.

According to the present invention, the fluorescence spectrum of the DNA-stabilized metal cluster-based nucleic acid-sensor comprises at least two peaks, in particular at least two excitation maxima and at least two emission maxima, in particular in the visible and/or near infrared (NIR) spectrum.

In a particularly preferred embodiment of the present invention, the wavelengths of the at least two peaks, in particular the wavelengths of at least two excitation maxima and of at least two emission maxima, in particular in the visible and/or near infrared (NIR) spectrum, are at least 40 nm, preferably at least 45 nm, preferably at least 50 nm, preferably at least 55 nm, preferably at least 60 nm, preferably at least 65 nm, preferably at least 70 nm, preferably at least 75 nm, preferably at least 80 nm, apart from one another.

In a preferred embodiment, in the "ready-to-use" state, the DNA-stabilized metal cluster-based nucleic acid-sensor according to the present invention has a fluorescence spectrum with an excitation wavelength in the range of 350 to 550 nm, preferably 400 to 520 nm, preferably 450 to 480 nm, and an emission wavelength in the range of 450 to 650 nm, preferably 500 to 600 nm, preferably 530 to 570 nm.

Preferably, in the "ready-to-use" state, the DNA-stabilized metal cluster-based nucleic acid-sensor according to the present invention has a fluorescence spectrum with an excitation wavelength in the range of 450 to 750 nm, preferably 500 to 650 nm, preferably 550 to 600, and an emission wavelength in the range of 500 to 900 nm, preferably 550 to 700 nm, preferably 620 to 650 nm.

In a preferred embodiment of the present invention, the DNA-stabilized metal cluster-based nucleic acid-sensor according to the present invention has a fluorescence spectrum with i) a first excitation wavelength in the range of 350 to 550 nm, preferably 400 to 520 nm, preferably 450 to 480 nm, and a first emission wavelength in the range of 450 to 650 nm, preferably 500 to 600 nm, preferably 530 to 570 nm as well as ii) a second excitation wavelength in the range of 450 to 750 nm, preferably 500 to 650 nm, preferably 550 to 600, and a second emission wavelength in the range of 500 to 900 nm, preferably 550 to 700 nm, preferably 620 to 650 nm.

According to a particularly preferred embodiment of the present invention, in the "ready-to-use" state, the DNA-stabilized metal cluster-based nucleic acid-sensor according to the present invention has a fluorescence spectrum with i) a first excitation wavelength in the range of 400 to 520 nm, preferably 450 to 480 nm, and a first emission wavelength in the range of 500 to 600 nm, preferably 530 to 570 nm, as well as ii) a second excitation wavelength in the range of 550 to 700 nm, preferably 620 to 650 nm, and a second emission wavelength in the range of 500 to 900 nm, preferably 550 to 700 nm, preferably 620 to 650 nm.

Accordingly, the fluorescence spectrum of the DNA-stabilized metal cluster-based nucleic acid-sensor according to the present invention is characterized by two emission maxima. Particularly preferred, the fluorescence spectrum of the DNA-stabilized metal cluster-based nucleic acid-sensor according to the present invention is characterized by a first emission maximum in the range of 450 to 650 nm, preferably 500 to 600 nm, preferably 530 to 570 nm, and a second emission maximum in the range of 500 to 900 nm, preferably 550 to 700 nm, preferably 620 to 650 nm.

In a preferred embodiment of the present invention, the DNA-stabilized metal cluster-based nucleic acid-sensor has a limit of detection of at least 250 nM, preferably at least 225 nM, preferably at least 200 nM, preferably at least 175 nM, preferably at least 150 nM, preferably at least 125 nM, preferably at least 100 nM, preferably at least 175 nM, preferably at least 50 nM, preferably at least 25 nM, preferably at least 10 nM

According to the present invention, the DNA-stabilized metal cluster-based nucleic acid-sensor is characterized by a high specificity for the target nucleotide sequence. Particularly preferred, one mismatch between the at least one target-recognition nucleotide sequence of the DNA-stabilized metal cluster-based nucleic acid-sensor and the target nucleotide sequence reduces the read-out signal by at least 50%, preferably at least 55%, preferably at least 60%, preferably at least 65%, preferably at least 70%, preferably at least 75%.

In a preferred embodiment of the present invention, the DNA-stabilized metal cluster-based nucleic acid-sensor comprises the nucleotide sequence of **SEQ ID No. 1**, preferably consists thereof. Preferably, the DNA-stabilized metal cluster-based nucleic acid-sensor comprises a nucleotide sequence having at least 70%, preferably at least 75%, preferably at least 80%, preferably at least 85%, preferably at least 90%, preferably at least 95%, preferably at least 98%, preferably at least 99%, sequence identity with the nucleotide sequence of **SEQ ID No. 1.**

In a further preferred embodiment of the present invention, the DNA-stabilized metal cluster-based nucleic acid-sensor comprises the nucleotide sequence of **SEQ ID No. 2**, preferably consists thereof. Preferably, the DNA-stabilized metal cluster-based nucleic acid-sensor comprises a nucleotide sequence having at least 70%, preferably at least 75%, preferably at least 80%, preferably at least 85%, preferably at least 90%, preferably at least 95%, preferably at least 98%, preferably at least 99%, sequence identity with the nucleotide sequence of **SEQ ID No. 2.**

According to a preferred embodiment of the present invention, the DNA-stabilized metal cluster-based nucleic acid-sensor comprises the nucleotide sequence of **SEQ ID No. 3**, preferably consists thereof. Preferably, the DNA-stabilized metal cluster-based nucleic acid-sensor comprises a nucleotide sequence having at least 70%, preferably at least 75%, preferably at least 80%, preferably at least 85%, preferably at least 90%, preferably at least 95%, preferably at least 98%, preferably at least 99%, sequence identity with the nucleotide sequence of **SEQ ID No. 3.**

According to preferred embodiment, the DNA-stabilized metal cluster-based nucleic acid-sensor comprises the nucleotide sequence of **SEQ ID No. 4.**

In another preferred embodiment of the present invention, the DNA-stabilized metal cluster-based nucleic acid-sensor comprises the nucleotide sequence of **SEQ ID No. 5.**

The present invention further pertains to a method for detecting a target polynucleotide, comprising the steps:
i) providing a DNA-stabilized metal cluster-based nucleic acid-sensor according to the present invention,
ii) proving a sample comprising the target polynucleotide,
iii) mixing the nucleic acid-sensor with the target polynucleotide,
iv) measuring the intensities of two fluorescence maxima of the nucleic acid-sensor.

In a preferred embodiment of the present invention, the method further comprises:
v) calculating the concentration of the target polynucleotide from the ratio of intensities of two fluorescence maxima of the nucleic acid-sensor measured in step iv).

Thus, in this particular embodiment of the present invention, the method for detecting a target polynucleotide is a method for detecting and quantifying a target polynucleotide.

In a particularly preferred embodiment of the present invention, the DNA-stabilized metal cluster-based nucleic acid-sensor is a DNA-stabilized metal cluster-based DNA/RNA-sensor.

In the context of the present invention, the expression "DNA-stabilized metal cluster-based nucleic acid-sensor" is used for a sensor molecule suitable for specifically detecting target nucleic acid molecules, in particular target DNA molecules and/or RNA molecules, wherein the nucleic acid sensor comprises a DNA-stabilized metal cluster, in particular a silver quantum cluster (AgQC).

The expression "template nucleotide sequence for silver quantum cluster (AgQC) formation" is used herein for a nucleotide sequence suitable for binding, in particular suitable for complexing, silver quantum clusters.

In the context of the present invention, a "target-recognition nucleotide sequence" is a nucleotide sequence which is suitable to specifically bind to a target nucleotide sequence, in particular to at least partially hybridize with a target nucleotide sequence. In this regard, the "target-recognition nucleotide sequence" can either be a single continuous nucleotide sequence or can be formed by two or more partial target-recognition nucleotide sequences which together bind to a target nucleotide sequence, in particular at least partially hybridize with a target nucleotide sequence.

A "nucleotide sequence for intra-molecular hybridization" designates a nucleotide sequence which causes intra-molecular hybridization of particular nucleotide sequences within the nucleic acid-sensor according to the present invention. Preferably, the "nucleotide sequence for intra-molecular hybridization" comprises nucleotide sequence sections which are of a certain length, which are complementary to each other and which are sufficiently spaced apart from one another to result in hybridization of the nucleotide sequences sections. In a particularly preferred embodiment of the present invention, the "nucleotide sequence for intra-molecular hybridization" causes the formation of a stem-loop structure within the nucleic acid-sensor. According to this particularly preferred embodiment of the present invention, the "nucleotide sequence for intra-molecular hybridization" is a "nucleotide sequence for intra-molecular stem-loop formation". In a particular embodiment of the present invention, the nucleic acid-sensor comprises at least two, preferably two, "nucleotide sequence for intra-molecular hybridization". These "nucleotide sequences for intra-molecular hybridization" are preferably contained within the "template nucleotide sequence for silver quantum cluster (AgQC) formation".

According to the present invention, the "template nucleotide sequence for silver quantum cluster (AgQC) formation", the "target-recognition nucleotide sequence", and the "nucleotide sequence for intra-molecular hybridization" can either be completely separate nucleotide sequences, in particular consecutive nucleotide sequences located on a single nucleotide strand or can be partially overlapping nucleotide sequences. For example, a particular section of the "target-recognition nucleotide sequence" can be partially overlapping with the "nucleotide sequence for intra-molecular hybridization". In particular, certain nucleotides in the 5'- and/or 3'-region can function in target-recognition and also participate in the intra-molecular hybridization.

The expression "target polynucleotide sequence" designates a nucleic acid of interest, in particular a DNA or RNA molecule of interest, characterized by a specific nucleotide sequence.

In the context of the present invention, the term "a" is meant to include the meaning of "one" or "one or more". The term "comprising" preferably has the meaning of "containing" or "including", meaning including the specifically identified elements without excluding the presence of further elements. However, in a preferred embodiment, the term "comprising" is also understood to have the meaning of "consisting essentially of' and in a further preferred embodiment the meaning of "consisting of'.

The expression "and/or" is used herein to express the disclosure of any combination of individual elements connected within a listing. Solely for illustrative purpose, the expression "A, B, and/or C" can mean A individually, B individually, C individually, (A and B), (B and C), (A and C), and (A, B and C).

Further preferred embodiments of the present invention are subject of the subclaims. The invention is described in the following by way of examples and the accompanying figures.

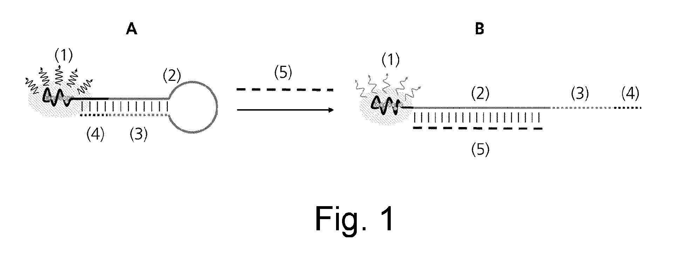

**Fig. 1** schematically shows a possible mode of action for target recognition by a DNA-stabilized metal cluster-based nucleic acid-sensor according to the present invention. In the closed "ready to use" state (**A**), a stem-loop structure is formed by hybridization between a template nucleotide sequence for silver quantum cluster (AgQC) formation (1), a nucleotide sequence which is partially complementary to the 3'-end of the template sequence for silver quantum cluster (AgQC) formation (4) as well as between a target-recognition nucleotide sequence (2) and a nucleotide sequence which is partially complementary to the 5'-end of the target-recognition sequence (3). Upon target polynucleotide (5) recognition, the closed "ready to use" state transitions into an open state (**B**). In this process, the target-recognition nucleotide sequence (2) hybridizes with the target polynucleotide (5), thereby opening the stem-loop structure of the nucleic acid-sensor and dissolving the intra-molecular hybridization. Within the closed state (**A**), the nucleotide sequence which is partially complementary to the 3'-end of the template sequence for silver quantum cluster (AgQC) formation (4) interferes with the template nucleotide sequence for silver quantum cluster (AgQC) formation (1). The transition from the closed state (**A**) to the open state (**B**) resolves the structure interference between these two nucleotide sequences by separation thereof. This results in a measurable shift in the fluorescence intensity of the two characteristic peaks in the fluorescence spectrum of the DNA-based sensors.

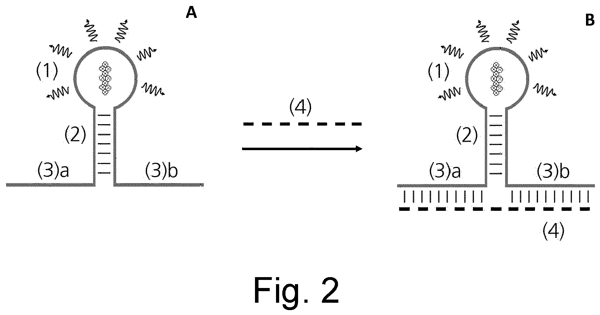

A further embodiment of the DNA-stabilized metal cluster-based nucleic acid-sensor according to the present invention is shown in **Fig. 2****.** In this embodiment, the template nucleotide sequence for silver quantum cluster (AgQC) formation (1) contains a stem-loop structure of the nucleic acid-sensor. The binding of the AgQC ligand in the stem-loop structure results in an enhanced stability. This allows for the design to be less toxic and more stable in more aggressive environments. On the other side, the target-recognition sequence (3) is less protected in this embodiment. The stem-loop structure is stabilized by two complementary sequences for intra-molecular hybridization (2). In the embodiment shown in **Fig. 2**, the target-recognition nucleotide sequence (3) is composed of two partial target-recognition nucleotide sequence (3a, 3b) which are located in the 5'- and 3'-region of the DNA-based sensor, respectively.

In the "ready to use" state (**A**), the two complementary sequences for intra-molecular hybridization (2) stabilize the stem-loop structure containing the fluorescent AgQC. Both parts of the target-recognition sequences (3a, 3b) are available for hybridization to a complementary target nucleotide sequence (4). Upon hybridization (**B**), the stabilized T-shaped DNA structure triggers a change to the quantum cluster's environment, such that the fluorescence intensities of two characteristic wavelengths in the emission spectrum of the DNA-based sensor change significantly.

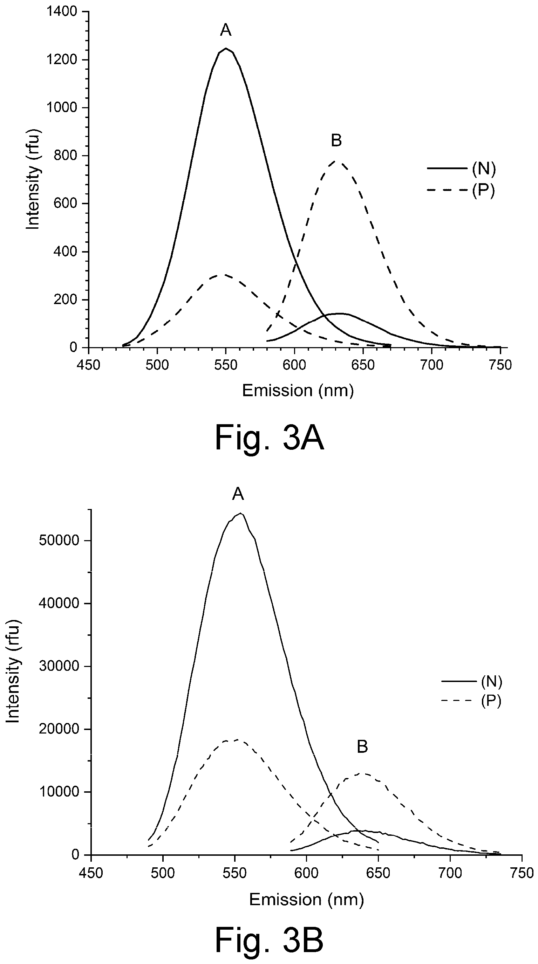

**Fig. 3A** illustrates the fluorescence spectrum of a DNA-stabilized metal cluster-based nucleic acid-sensor configured as given in **Fig. 1** in the absence (N, solid) and presence (P, dashed) of the randomly generated target polynucleotide-1 with 22 nucleotides. In **Fig. 3B**, the fluorescence spectrum of a DNA-stabilized metal cluster-based nucleic acid-sensor configured as given in **Fig. 1** in the absence (N, solid) and presence (P, dashed) of the *mecA* gene as target polynucleotide is shown. (N) = Negative (nucleic acid-sensor in the absence of the respective target polynucleotide); (P) = Positive (nucleic acid-sensor in the presence of the respective target polynucleotide). A = emission maximum (λem = 550 ± 5 nm); B = emission maximum (λem = 635 ± 5 nm).

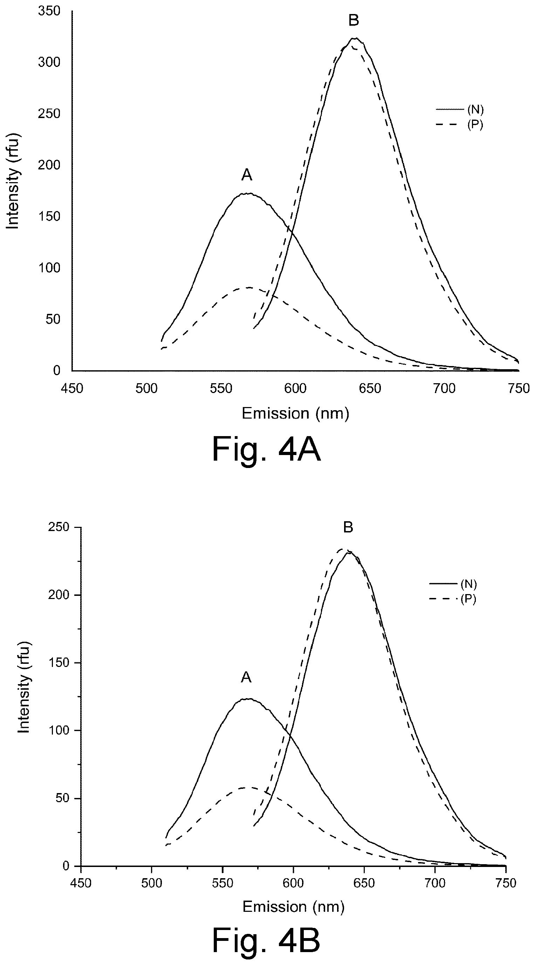

In **Fig. 4A**, the fluorescence spectrum of a DNA-stabilized metal cluster-based nucleic acid-sensor configured as given in **Fig. 2** in the absence (N, solid) and presence (P, dashed) of the randomly generated target polynucleotide-1 with 22 nucleotides is shown. **Fig. 4B** illustrates the fluorescence spectrum of a DNA-stabilized metal cluster-based nucleic acid-sensor configured as given in **Fig. 2** in the absence (N, solid) and presence (P, dashed) of a 30-nucleotide fragment of the *bla-TEM* gene of *E. coli* as target polynucleotide. (N) = Negative (nucleic acid-sensor in the absence of the respective target polynucleotide); (P) = Positive (nucleic acid-sensor in the presence of the respective target polynucleotide). A = emission maximum (λem = 565 ± 5 nm); B = emission maximum (λem = 630 ± 5 nm).

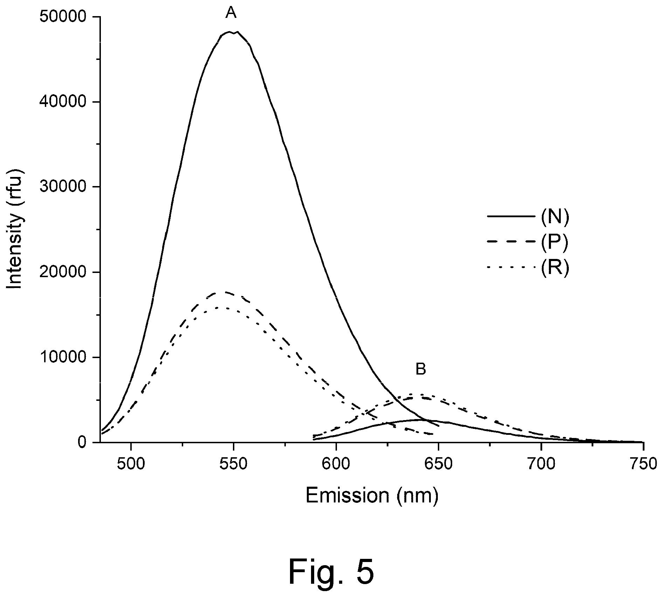

**Fig. 5** illustrates the fluorescence spectrum of a DNA-stabilized metal cluster-based nucleic acid-sensor configured as given in **Fig. 1** in the absence (N, solid) and presence (P, dashed) of an RNA genome from SARS-CoV-2 Delta variant (B. 1. 617.2) as target polynucleotide. As a reference (R, dotted), the fluorescence spectrum of the previous mentioned DNA-stabilized metal cluster-based nucleic acid-sensor in presence of the respective DNA sequence of the genome from SARS-CoV-2 Delta variant (B.1.617.2) is shown. (N) = Negative (nucleic acid-sensor in the absence of the respective target polynucleotide); (P) = Positive (nucleic acid-sensor in the presence of the respective RNA target polynucleotide); (R) = Reference (nucleic acid-sensor in the presence of the respective DNA target polynucleotide). A = emission maximum (λem = 550 ± 5 nm); B = emission maximum (λem = 635 ± 5 nm).

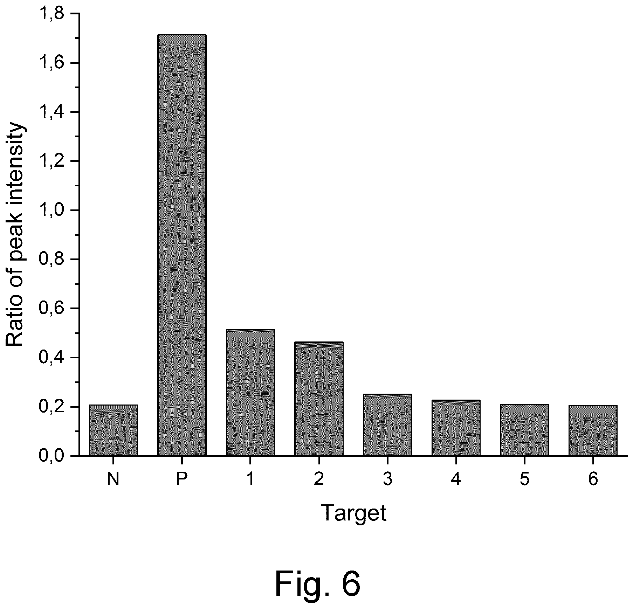

**Fig. 6** shows the specificity of a nucleic acid-sensor according to the present invention. It is evident that already a mismatch in one base between the target polynucleotide and the target-recognition nucleotide sequence results in a more than 70 % reduced read-out signal. N = Negative (nucleic acid-sensor in the absence of target polynucleotide-1); P = Positive (nucleic acid-sensor in the presence of target polynucleotide-1); 1-6 = mismatch target polynucleotides with 1 to 6 non-complementary bases.

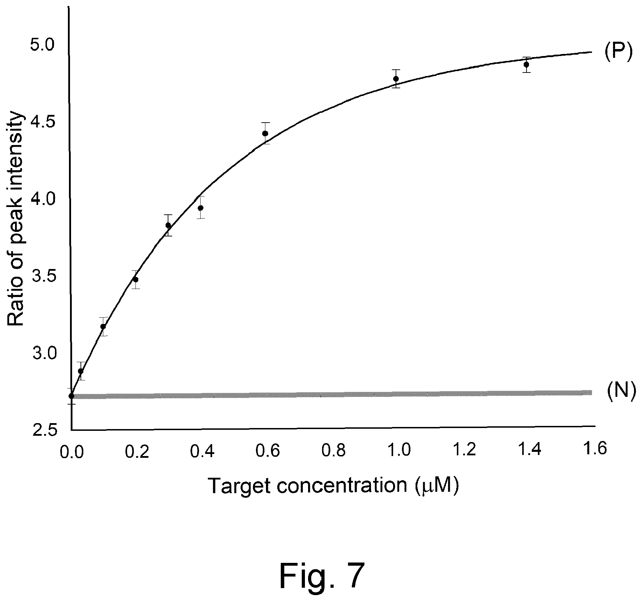

**Fig. 7** shows the measured ratio of emission intensities as a function of the concentration of target DNA material. The graph shows the combined results of two sequences used in calibration. Error bars represent the standard deviation in the repeated experiments (n = 5). The grey line is the baseline ratio in the absence of a target polynucleotide. The calibration was performed with a sensor concentration of 1.5 µM.

### Example 1: Preparation and quality control of a DNA-stabilized metal cluster-based nucleic acid-sensor

For the preparation of the sensor according to the present invention, an Ag-DNA synthesis is performed. The sensor DNA sequence is dissolved in ammonium acetate buffer solution, heated to 95 °C for 10 min to unfold, and incubated on ice for 5 min. Silver nitrate (AgNO₃) solution is added and the mixture is incubated at 4 °C for 30 min. Sodium borohydride (NaBH₄) is added for reduction and the mixture is incubated again for 3 hours at 4 °C. After a purification step, the sensor solution is ready for use and can be stored at 4 °C in the absence of light for several months.

For the application, the readiness for use and integrity of the sensor can be easily assessed at any time after the storage period. For this purpose, the intact fluorescence can be checked at the given excitation and emission wavelengths.

### Example 2: Application of a DNA-stabilized metal cluster-based nucleic acid-sensor

For detection of the target polynucleotide, the aqueous sample solution is mixed with the sensor solution and incubated for 2 hours at room temperature. After 2 hours, a calorimetric evaluation with the naked eye (with and without UV lamp) can already be carried out for high concentrations of the target polynucleotide. For a more sensitive detection, a simple measurement of the intensities of the given two fluorescence maxima is performed. The target concentration can then be easily determined from the ratio between the two intensities.

The ease of operation, good temperature stability of up to 60 °C and low photo bleaching allows even for the (non-professional) use outside a laboratory environment. Even for sensitive detection, all that is required is an excitation light source and a detector for the light intensity. Accordingly, even an application of the sensor at home and in remote areas is possible.

### Example 3: Detection of mecA gene (methicillin-resistant Staphylococcus aureus (MRSA))

The sensor was prepared according to the method described in **Example 1.** For the detection of the *mecA* gene, a final concentration of 2 µM sensor DNA material (**SEQ ID No. 1**: 5'*-TTCCCACCCACCCCGGCCCGTTAAAATCGATGGTAAAGGTTGGCTCGATTTTAACG-*3'), is mixed with 20 µM AgNO₃ and 10 µM NaBH₄ in NH₄OAC buffer solution (20 mM, pH: 7.0). For purification and buffer exchange of the synthesized sensor, the sensor solution is loaded onto a 3 kDa centrifuge filter and washed twice with NH₄OAC buffer solution (100 mM, pH: 7.0). After eluting the purified sample from the filter, NH₄OAC buffer solution (100 mM, pH: 7.0) is added up to the original sample volume.

For detection of the target polynucleotide (5'*-GCCAACCTTTACCATCGATTTT*-3')*,* 5 µl of purified sensor solution is mixed with 1 µl of the aqueous sample solution and 4 µl of bi-distilled water, followed by incubation at room temperature for 2 hours. Subsequently, a simple measurement of the intensities of the two fluorescence maxima (green-yellow: λex/ λem = (465 ± 5 nm) / (550 ± 5 nm) and red: λex/ λem = (575 ± 5 nm) / (635 ± 5 nm)) is performed. In this example, the fluorescence was collected through Tecan Spark spectrometer (see **Fig. 3B**). The *mecA* gene concentration is determined from the ratio between the two fluorescence maxima.

### Example 4: Detection of bla-TEM gene (ampicillin-resistant Escherichia coli)

The sensor was prepared according to the method described in **Example 1.** For the detection of *bla-TEM* gene, 1.5 µM of sensor DNA material (**SEQ ID No. 2:** 5'*-GTTATGGCAGCACTGCACTTACCTCCCCCCCCCCCAGGTAAGTATAATTCTCTTACT-*3') is mixed with 10.5 µM AgNO₃ and 2.5 µM NaBH₄ in NH₄OAC buffer solution (20 mM, pH: 7.0). After synthesis, the solution is loaded onto a 3 kDa centrifuge filter. Using these filters, the sample is washed twice with the NH₄OAC buffer solution as used before.

For detection, the sensor is mixed with the target polynucleotide (5'-*ACAGTAAGAGAATTATGCAGTGCTGCCATAAC*-3') in equal concentrations in a volume of 40 µl. Measurement of the fluorescence intensities can be performed after 1 hour incubation. In this example, the fluorescence was collected through Cary Eclipse fluorimeter (Varian Medical systems) (see **Fig. 4B**). The concentration of the *bla-TEM* gene fragment can be determined from the ratio of the two fluorescence maxima (green-yellow: λex/ λem = (475 ± 5 nm) / (565 ± 5 nm) and red: λex/ λem = (575 ± 5 nm) / (630 ± 5 nm)).

### Example 5: Detection of virus RNA from SARS-CoV-2 Delta variant (B. 1.617.2)

The sensor was prepared according to the method described in **Example 1.** For the detection of the virus RNA, a final concentration of 20 µM sensor DNA material (**SEQ ID No. 3**: 5'*-TTCCCACCCACCCCGGCCCGTTCGGGAATTATAATTACCACCAACCAATTCCCGAACG-*3') is mixed with 200 µM AgNO₃ and 100 µM NaBH₄ in NH₄OAC buffer solution (20 mM, pH: 7.0). For purification and buffer exchange of the synthesized sensor, the sensor solution is loaded onto a 3 kDa centrifuge filter and washed twice with NH₄OAC buffer solution (20 mM, pH: 7.0). After eluting the purified sample from the filter, NH₄OAC buffer solution (20 mM, pH: 7.0) is added up to the original sample volume.

For detection, the sensor is mixed with the target polynucleotide (5'-*GGUUGGUGGUAAUUAUAAUUCCCG-*3') in equal concentrations in a volume of 100 µl, followed by incubation at room temperature for 2 hours. Subsequently, a simple measurement of the intensities of the two fluorescence maxima (green-yellow: λex/ λem = (465 ± 5 nm) / (550 ± 5 nm) and red: λex/ λem = (575 ± 5 nm) / (635 ± 5 nm)) is performed. In this example, the fluorescence was collected through Tecan Spark spectrometer (see **Fig. 5**). The SARS-CoV-2 virus RNA concentration is determined from the ratio between the two fluorescence maxima.

## Claims

1. A DNA-stabilized metal cluster-based nucleic acid-sensor comprising:
a) at least one template nucleotide sequence for silver quantum cluster (AgQC) formation,
b) at least one target-recognition nucleotide sequence, and
c) at least one nucleotide sequence for intra-molecular hybridization,
wherein the DNA-stabilized metal cluster-based nucleic acid-sensor comprises a stem-loop structure in the absence of a target nucleotide sequence.

2. The DNA-stabilized metal cluster-based nucleic acid-sensor according to claim 1, wherein the nucleotide sequences of a), b), and c) are part of a single nucleotide strand.

3. The DNA-stabilized metal cluster-based nucleic acid-sensor according to claim 2, wherein the single nucleotide strand has a length of 35 to 80 nucleotides, preferably 45 to 65 nucleotides, preferably 50 to 60 nucleotides.

4. The DNA-stabilized metal cluster-based nucleic acid-sensor according to any one of the preceding claims, wherein the at least one target-recognition nucleotide sequence has a length of 15 to 50 nucleotides, preferably 20 to 40 nucleotides, preferably 25 to 35 nucleotides.

5. The DNA-stabilized metal cluster-based nucleic acid-sensor according to any one of the preceding claims, wherein sensor comprises at least one silver quantum cluster (AgQC).

6. The DNA-stabilized metal cluster-based nucleic acid-sensor according to any one of the preceding claims, wherein the at least one nucleotide sequence for intra-molecular hybridization comprises:
i) at least one nucleotide sequence which is partially complementary to the target-recognition sequence, and
ii) at least one nucleotide sequence which is partially complementary to the template sequence for silver quantum cluster (AgQC) formation.

7. The DNA-stabilized metal cluster-based nucleic acid-sensor according to any one of the claims 1 to 5, wherein the at least one target-recognition nucleotide sequence of the DNA-stabilized metal cluster-based nucleic acid-sensor is composed of at least two partial target-recognition nucleotide sequences.

8. The DNA-stabilized metal cluster-based nucleic acid-sensor according to claim 7, wherein at least one partial target-recognition nucleotide sequence is located in the 5'-region of the single nucleotide strand and at least one partial target-recognition nucleotide sequence is located in the 3'-region of a single nucleotide strand.

9. The DNA-stabilized metal cluster-based nucleic acid-sensor according to claim 7 or 8, wherein the at least one template nucleotide sequence for silver quantum cluster (AgQC) formation comprises a stem-loop structure.

10. The DNA-stabilized metal cluster-based nucleic acid-sensor according to any one of the preceding claims, wherein the sensor comprises at least two excitation maxima and at least two emission maxima, in the visible and/or near infrared (NIR) spectrum which are at least 40 nm, preferably at least 50 nm, preferably at least 60 nm, preferably at least 70 nm, preferably at least 80 nm, apart from one another.

11. The DNA-stabilized metal cluster-based nucleic acid-sensor according to any one of the preceding claims, wherein the sensor has a fluorescence spectrum with i) a first excitation wavelength in the range of 350 to 550 nm, preferably 400 to 520 nm, preferably 450 to 480 nm, and a first emission wavelength in the range of 450 to 650 nm, preferably 500 to 600 nm, preferably 530 to 570 nm, as well as ii) a second excitation wavelength in the range of 450 to 750 nm, preferably 500 to 650 nm, preferably 550 to 600 nm, and a second emission wavelength in the range of 500 to 900 nm, preferably 550 to 700 nm, preferably 620 to 650 nm.

12. The DNA-stabilized metal cluster-based nucleic acid-sensor according to any one of the preceding claims, wherein the sensor has a limit of detection of at least 10 nM, preferably at least 50 nM, preferably at least 100 nM, preferably at least 200 nM, preferably at least 250 nM.

13. The DNA-stabilized metal cluster-based nucleic acid-sensor according to any one of the preceding claims, comprising a nucleotide sequence as defined in SEQ ID No. 1, SEQ ID No. 2, or SEQ ID No. 3.

14. A method for detecting a target polynucleotide, comprising the steps:
i) providing a DNA-stabilized metal cluster-based nucleic acid-sensor according to any one of claims 1 to 13,
ii) providing a sample comprising the target polynucleotide,
iii) mixing the sensor with the target polynucleotide,
iv) measuring the intensities of two fluorescence maxima of the sensor.

15. The method according to claim 14, wherein the nucleic acid-sensor is used in a concentration of 0.1 to 10 µM, preferably 1 to 5 µM.

16. The method according to claim 14 or 15, further comprising:
v) calculating the concentration of the target polynucleotide from the ratio of intensities of two fluorescence maxima of the nucleic acid-sensor measured in step iv).
